Europäisches Patentamt

European Patent Office

Office européen des brevets

(11) Publication number: **0 279 010**
**A2**

# (12) EUROPEAN PATENT APPLICATION

(21) Application number: **87108895.1**

(22) Date of filing: **22.06.87**

(51) Int. Cl.⁴: **A61K 35/78** , C07G 17/00

(30) Priority: **19.02.87 JP 36521/87**

(43) Date of publication of application:
**24.08.88 Bulletin 88/34**

(84) Designated Contracting States:
**AT CH DE ES FR GB IT LI NL**

(71) Applicant: **Kao Corporation**
**1-14-10, Nihonbashi Kayaba-cho**
**Chuo-ku Tokyo(JP)**

(72) Inventor: **Kitahara, Takashi**
**396-12, Mine-machi**
**Utsunomiya-shi Tochigi-ken(JP)**
Inventor: **Yoshida, Eisaku**
**2606-6, Akabane Ichikal-machi**
**Haga-gun Tochigi-ken(JP)**
Inventor: **Akatsu, Mitsuhiro**
**2992-56, Ishii-machi**
**Utsunomiya-shi Tochigi-ken(JP)**
Inventor: **Jokura, Yoji**
**396-12, Mine-machi**
**Utsunomiya-shi Tochigi-ken(JP)**
Inventor: **Hattori, Michihiro**
**420-34, Hiramatsu-cho**
**Utsunomiya-shi Tochigi-ken(JP)**
Inventor: **Takaishi, Naotake**
**1022-24, Himuro-machi**
**Utsunomiya-shi Tochigi-ken(JP)**

(74) Representative: **Wächtershäuser, Günter, Dr.**
**Tal 29**
**D-8000 München 2(DE)**

(54) **5-alpha-reductase inhibitor.**

(57) A $5\alpha$-reductase inhibitor comprising as an effective component an extract from leaves and/or pericarps of a walnut.

The inhibitor exhibits high potent $5\alpha$-reductase inhibiting activity and high safety, and is thus effective in curing and preventing diseases caused by testosterone, e.g. male-pattern alopecia, hirsutism, prostate cancer and the like.

EP 0 279 010 A2

## 5α-REDUCTASE INHIBITOR

### BACKGROUND OF THE INVENTION

1) Field of the Invention

This invention relates to a 5α-reductase inhibitor and more particularly, to 5α-reductase inhibitor comprising as an effective component an extract from walnut leaves and/or pericarps.

2) Description of the Prior Art

In hair troubles such as male-pattern alopecia or hirsutism, hindradenitis suppurativa whose cause is considered to depend partially on the functional disorder of the apocrine gland, or diseases such as prostatomegaly and prostate cancer, androgen is considered to be a critical cause or an exacerbating factor. Accordingly, a variety of anti-androgen have been in use for curing these diseases. The anti-androgen is known to have such mechanisms of action including a 5α-reductase activity inhibiting action in which testosterone, one of androgens, is inhibited from being reduced in these target organs into more active dihydrotestosterone by the action of 5α-reductase and the action of inhibiting binding with an receptor in which testosterone or the produced dihydrotestosterone is inhibited from binding with an acceptor within target cells. Currently employed curing medicines as anti-androgen agents include oxendrone, chloromadinone acetate, 11-α-hydroxyprogesterone, 4-androstene-3-on-17β-carboxylic acid, cyproterone acetate and the like. All of these compounds have either of or both the 5α-reductase inhibiting action and/or the inhibiting action of binding with a receptor, thereby inhibiting the action of androgen.

However, these anti-androgens are derivatives of steroid hormone. When they are dosed to a body over a long term, there is the safety problem that because of the hormonal action of these compounds themselves or their metabolites, side effects may arise.

Accordingly, there is a demand of development of a novel anti-androgen which is completely free of any safety problems.

### SUMMARY OF THE INVENTION

The present inventors made intensive studies in order to solve the above problem. As a result, it was found that an extract from walnut leaves and/or pericarps had a potent 5α-reductase inhibiting effect. The present invention was accomplished based on the above finding.

An extract from walnut leaves has been long used not only as a hairdye, but also as a hair tonic or a hair growth agent (Genshoku Yakuyo Shokubutsu Zukan, Published by Hoikusha). However, the crude extract was not satisfactory with respect to the effect as the hair tonic or hair growth agent and the mechanism of the action of the extract was not known. Our investigations revealed that the action depends on the 5α-reductase inhibiting substance contained in an extract of walnut leaves.

### DETAILED DESCRIPTION OF THE INVENTION AND PREFERRED EMBODIMENTS

The extract from the walnut leaves and/or pericarps (hereinafter referred to simply as walnut extract), which is an effective component of the 5α-reductase inhibitor according to the invention, is obtained, for example, as follows: starting walnut leaves and/or pericarps (hereinafter referred to simply as starting material) are, preferably after drying and grinding, extracted by immersion in a solvent at normal temperatures or elevated temperature, or are extracted by the use of an extractor such as the Soxhlet's extractor.

The solvent for the extraction may be water or organic solvents, of which organic solvents are preferred.

Examples of the solvents include polyhydric alcohols such as glycerine, polyethylene glycol, propylene glycol and the like, mixed solutions of polyhydric alcohols and water (preferably 5 to 30% aqueous solutions), aqueous solutions of surface active agents such as anionic, nonionic and amphoteric surface active agents, alcohols such as methanol, ethanol, butanol and the like, mixtures of alcohols and water (10 - 90%, preferably 20 - 80%, aqueous solutions), hydrocarbons such as hexane, benzene, toluene, xylene, petroleum ehter and the like, halogenated hydrocarbons such as dichloromethane, 1,2-dichloroethane and the like, ethers such as diethyl ether, tetrahydrofuran and the like, esters such as ethyl acetate, isopropyl myristate and the like, and various mineral, plant and animal oils such as liquid paraffin, soybean oil, sesame oil, and the like.

Of these, preferable solvents include a 10 to 25% aqueous solution of polyethylene glycol (molecular weight: 400 to 1000), butanol, soybean oil, sesame oil, hydrous alcohols such as 20 to 80% hydrous methanol and ethanol, hydrocarbons such as benzene, toluene, petroleum ether and the like, dimethyl sulfoxide, isopropyl myristate and the like. Most preferably, hydrocarbons such as benzene, toluene, petroleum ether and the like, and hydrous lower alcohols such as hydrous methanol and hydrous ethanol are mentioned.

Preferable methods for the extraction include a method in which the starting material is ground and extracted with a solvent such as a hydrocarbon and a hydrous lower alcohol; a method in which ground pieces or powder of the starting material are extracted with a hydrous lower alcohol, the resultant extraction phase is subjected to liquid-liquid extraction using a hydrocarbon, and distilling the solvent from the hydrocarbon phase to which the active ingredient is transferred; and a method in which an extraction phase is treated with a resin such as polyamide resin to allow an active ingredient to be adsorbed thereon, extracting the active ingredient with methanol, ethanol, acetone or their mixed solution with water, and distilling off the solvent.

For the extraction by immersion, when a polyethylene glycol aqueous solution is used as the solvent, the time for the immersion may be 4 to 8 days at 20 to 50°C. Likewise, the immersion time may be 4 to 8 days at 15 to 40°C for butanol, 2 to 3 days at 40 to 80°C for soybean or sesame oil, and 4 to 8 days at 15 to 40°C for benzene, toluene, methanol, ethanol, dimethyl sulfoxide, petroleum ether or isopropyl myristate. The thus obtained extract may be used, as it is, as an effective component for the 5α-reductase inhibitor. Alternatively, the extract may be used after fractionation with a suitable isolation means including, for example, gel filtration, silica gel column chromatography, reverse or normal phase high performance liquid chromatography, thereby obtaining a fraction of higher activity.

According to the above methods, not only a walnut extract of high activity is obtained, but also the problem of a known extract which has an objectionable color or odor can be solved.

The thus obtained walnut extract has the 5α-reductase inhibiting activity and does not involve any safety problem as will be appreciated from the fact that when sample 1 of the invention obtained in Reference 1 was dosed to mice at 10 g/kg, any mice did not die.

The 5α-reductase inhibitor comprising the walnut extract as an effective component is useful in safely and effectively curing various diseases which are caused or exacerbated by the action of testosterone, e.g. hindradenitis suppurativa, axillary odor, hirsutism, prostatomegaly and prostate cancer. Alternatively, the inhibitor may be also used for curing male-pattern alopecia. Depending upon the purpose, the 5α-reductase inhibitor may be dosed generally or locally, or orally or parenterally. The dosage may vary depending upon the age, body weight, sex, symptoms, curing effect, manner of the dose, and curing time. For the curing and/or prevention of prostatomegaly, it is general to dose it orally in an amount of 1 mg to 1 g, preferably 20 mg to 200 mg for an adult at one time and one to several times in a day. Alternatively, it may be parenterally dosed one to several times in a day in an amount of 100 μg to 200 mg, preferably 1 mg to 10 mg, per dosage for an adult. For the curing and/or prevention of alopecia, 10 μg to 100 mg, preferably 50 μg to 10 mg, at one time for an adult is orally dosed one to several times in a day. Since the dosage may vary depending upon various conditions, smaller amounts may be sufficient or larger amounts may be required.

When the 5α-reductase inhibitor of the invention is used as a oral medicine, the extract may be dosed as it is, or may be formed into a desired preparation such as a tablet, a powder, a granule, a capsule, a liquid or the like, in order to enhance the efficacy. For the solid preparations, there may be used inert diluents such as lactose, mannitol, dextrose, hydroxypropyl cellulose, fine crystalline cellulose, starch polyvinylpyrrolidone, magnesium metasilicoaluminate and the like, lubricants such as magnesium stearate, and disintegrators such as calcium cellulose gluconate. If necessary, the tablet or pill may be coated with one or more film layers of stomachically or intestinally soluble substances such as white sugar, gelatine, hydroxypropyl cellulose, hydroxypropyl methylcellulose, phthalates and the like.

Examples of liquid preparations for oral administration include an emulsion, a solution, a suspension, a syrup, an elixir and the like. For these liquid preparations, there may be comprise, aside from the effective ingredient and an inert diluent, adjuvants such as humectants, suspensing agents and the like, sweetenings, flavoring agents, aromatics, preservatives and the like.

Other preparations for oral administration include sprays.

Typical preparations which are used for parenteral administration are injections. For the preparation of an injection, the effective component of the invention is dissolved, suspended or emulsified in aqueous mediums such as distilled water for injection and physiological saline solution or in non-aqueous mediums such as propylene glycol, polyethylene glycol, plant oils such as olive oil, alcohols such as ethanol, non-aqueous mediums such as polysolvate 80, and the like. The injection may contain adjuvants such as presevatives, humectants, emulsifieres, dispersing agents, and the like. A sterilization means required for the injection may be filtration through a bacterium-retaining filter, incorporation of bactericides, irradiation, and the like. Alternatively, the injection may be formed as a sterile solid composition, which is subsequently used after dissolution in sterile water or a sterile solvent for injection.

Other preparations for parenteral administration include external solutions, endermic liniments such as an ointment, a lotion, a tonic, a spray, a suspension, an emulsion and the like, suppositories for intra-rectal administration, and pessaries for intra-vaginal administration. For the preparations for curing and preventing alopecia, lotions, tonics, sprays, solutions and ointments are preferred. These preparations may comprise, aside form the effective ingredient, distilled water, lower alcohols such as ethanol, higher alcohols such as cetanol, polyhydric alcohols such as polyethylene glycol, propylene glycol and the like, cellulose materials such as hydroxypropyl cellulose, animal, plant and synthetic oils and fats, vaseline, wax, silicones, surface active agents, diluents such as zinc oxide, and adjuvants such as humectants, suspending agents, aromatics, preservatives and the like.

The $5\alpha$-reductase inhibiting substance contained in the walnut extract according to the invention has not been identified yet. The active component is pronouncedly concentrated in a fraction soluble in organic solvents and can remarkably enhance the anti-androgenic action. Because of small degrees of coloration and odor, the substance can be readily applied to various cosmetics and medicines. Moreover, the $5\alpha$-reductase activity-inhibiting substance used in the present invention was confirmed to have a similar action to most of known $5\alpha$-reductase activity inhibiting substance, namely, an inhibiting action against a receptor for testosterone.

The $5\alpha$-reductase inhibitor of the invention exhibits high potent $5\alpha$-reductase inhibiting activity and high safety and is thus effective in curing and peventing diseases caused by testosterone, e.g. male-pattern alopecia, hindradenitis suppurativa, hirsutism, prostatomegaly and prostate cancer.

Examples and references are described.

**Reference 1**

Five hundred grams (on a dry basis) of leaves of an English walnut was ground, to which 2 liters of a 70% methanol aqueous solution was added, followed by heating to 50°C and shaking for 1 week. Thereafter, an extract was filtered to obtain a filtrate as sample liquid 1. An equal amount of toluene was added to the sample liquid 1 and subjected to liquid-liquid extraction for 4 days, followed by collecting the toluene phase and removal of the toluene by distillation under reduced pressure, thereby obtaining a solid matter as sample I.

**Reference 2**

Five hundred grams (on a dry basis) of pericarps of a Persia walnut was ground, to which 2 liters of toluene was added and shaked at room temperature for 1 week. Thereafter, the extract was filtered and the toluene was distilled off under reduced pressure to obtain sample II.

**Reference 3**

Five hundred grams (on a dry basis) of a Japanese wing nut was ground, to which 5 liters of benzene was added and shaked at room temperature for 1 week. The resultant extract was filtered and purified with the silica gel column chromatography, followed by distillation under reduced pressure for removal of the benzene, thereby obtaining a solid matter as sample III.

**Example 1**

(Inhibiting effect on 5α-reductase)

Male rats (weighing 300 - 450 g) were killed by cervical dislocation, followed by taking out of the prostate and homogenization with a 1:3 by volume of a 20 mM phosphate buffer (pH 7.5) containing 0.32 M of succrose. The mixture was centrifugated (140,000 g × 60 minutes) to obtain a precipitate, which was solubilized in a 40% glycerine-containing 10 mM phosphate buffer containing 5 mg/ml of digitonin and centrifugated (150,000 g × 60 minutes) to obtain a supernatant liquid as an enzyme solution. (See T. Liang et al, J. Biol. Chem., 256, 7998, 1981.) The measurement of an enzymatic activity was carried out as follows. 50 μl of the enzymatic solution, 0.25 μCi of [1,2,6,7,16,17-³H]-testosterone/10 μl of NADPH (50 μM), and specimens of various concentrations or a non-radioactive testosterone in a total amount of 500 μl, were incubated at 37°C for 1 hour. One milliliter of ethyl acetate was added to the mixture to stop the reaction. The ethyl acetate fraction was collected, from which the solvent was distilled off in an atmosphere of nitrogen, followed by re-dissolution in 50 μg of methanol. 20 μl of the solution was subjected to high performance liquid chromatography to measure the amounts of testosterone and dihydrotestosterone by the use of an UV detector and an isotope detector. An enzymatic inhibitory activity was calculated from the amounts of testosterone and dihydrotestosterone. The results are shown in Table 1. The results show that the extract of walnut leaves and/or pericarps has the 5α-reductase inhibiting effect and the active ingredient is concentrated in a fraction soluble in organic solvents.

## Table 1

| Specimen | Concentration (μg/ml) | Inhibiting Rate (%) |
|---|---|---|
| Sample 1* (solid matter after distillation of an extract) | 200 | 40 |
| | 100 | 29 |
| | 50 | 16 |
| Sample I | 50 | 95 |
| | 25 | 61 |
| | 10 | 35 |
| Sample II | 100 | 84 |
| | 50 | 57 |
| | 10 | 8 |
| Sample III | 25 | 75 |
| | 5 | 43 |

**Example 2**

By using sample I obtained in Reference 1, a hair tonic having a formulation shown in Table 2 was prepared so as to evaluate an influence on trichogram of the hair. Persons to be tested were 20 men, 30 to 35 years old, who suffered from falling out of the hair. The evaluation was effected as follows: the head was divided at center into left and right portions, one being applied with a hair tonic, the other being applied with a hair tonic base every day. At 0th day, one month, three months and 6 months after starting the test, three portions each with an area of $7 \times 7$ mm², which were kept apart 3 cm from center at the top of the head, were subjected to measurement of the trichogram, thereby determining a rate of telogen and the number of anagen in the hair group. For aging and the male-pattern alopecia, an increase in the rate of telogen and a decrease of the number of anagen are significantly recognized (Hattori et al: 85th Meeting of Japanese Skin Science Association, May 17, 1986, at Kyoto).

## Table 2

(wt%)

|  | Hair Tonic Base | Hair Tonic (Inventive Product) |
|---|---|---|
| Ethanol | 40.0 | 40.0 |
| Polyoxyethylene glycol hydrogenated castor oil | 0.4 | 0.4 |
| Methanol | 0.1 | 0.1 |
| Perfume | 0.1 | 0.1 |
| Sample I | - | 10.0 |
| Purified water | suitable amount | suitable amount |

The results are shown in Tables 3 and 4. At one month, no change is recognized between the hair tonic-treated sites and the base-treated sites, but the reduction in the rate of telogen at the hair tonic-treated sites is recognized. With respect to the number of anagen in the hair group, the rate of the hair groups consisting of one anagen decreases at the hair tonic-treated sites. As is inversely proportional to the above, the rate of the hair groups consisting of three anagens increases, from which the density of the hairs in the treated sites is found to increase.

## Table 3

| Treating Period (months) | Rate of Hairs at Resting Stage (%) | |
| --- | --- | --- |
| | Hair Tonic (Inventive Product) | Hair Tonic Base |
| 0 | 24 | 23 |
| 1 | 22 | 21 |
| 3 | 18 | 24 |
| 6 | 16 | 22 |

## Table 4

| Treatment | Rate Relative to Total Hair Groups (%) | | | | | | | |
| --- | --- | --- | --- | --- | --- | --- | --- | --- |
| | Hair Tonic (Inventive Product) | | | | Hair Tonic Base | | | |
| Treating Period (months) | 0 | 1 | 3 | 6 | 0 | 1 | 3 | 6 |
| Number of Hairs at Growth Stage in Hair Groups: | | | | | | | | |
| 1 | 38 | 37 | 33 | 28 | 37 | 36 | 38 | 35 |
| 2 | 48 | 49 | 45 | 46 | 45 | 47 | 44 | 47 |
| 3 | 12 | 11 | 15 | 18 | 14 | 15 | 13 | 14 |
| 4 | 1 | 2 | 3 | 4 | 2 | 1 | 3 | 2 |

**Example 3**

The sample III obtained in Reference 3 was evaluated with respect to the effect on the growth of human prostate cancer cells. The resluts are shown in Table 5. The medicine of the invention could significantly suppress the growth of the human prostate cancer cells.

### Table 5

|  | Growth Rate (%) |
|---|---|
| Base medium (MEM medium containing 10% vofoetal calf serum) | 100 |
| Base medium + Sample I (5 ppm) | 75 |
| Base medium + Sample I (50 ppm) | 20 |

**Example 4**

An ethanol solution containing 10% of sample III obtained in Reference 3 was prepared to evaluate the effect on axillary odor. The effect was evaluated according to the following criteria with respect to an increase or decrease in degree of the odor prior to or after the treatment. The test period was two weeks and the solution was applied twice a day.

(Evaluation Standards)

strong axillary odor   + +
Fair axillary odor   +
slight axillary odor   ±
no odor   -

As is appearant from Table 6, the solution containing the substance of the invention could reduce the axillary odor more than a 10% ethanol solution.

8

## Table 6

|  | Effective | Slightly Effective | Not Effective |
|---|---|---|---|
| Solution Base | 0* | 2 | 7 |
| Inventive Product | 1 | 5 | 4 |

*: Number of persons

## Claims

1. A 5α-reductase inhibitor comprising as an effective component an extract from leaves and/or pericarps of a walnut.

2. A 5α-reductase inhibitor according to Claim 1, wherein said extract from the leaves and/or pericarps of a walnut is an extract with an organic solvent.

9